# EUROPEAN PATENT APPLICATION

(11) **EP 4 775 130 A1**
(43) Date of publication of application: **15.07.2026**
(21) Application number: 24861938.9
(22) Date of filing: 02.09.2024
(51) Int. Cl.: A61B 3/13

(54) **PRISM SWITCHING MECHANISM AND OPHTHALMIC OPERATION MICROSCOPE**

(30) Priority: 06.09.2023 CN 202311148762
(71) Applicant: Towardpi (Beijing) Medical Technology Ltd., Beijing 102206 (CN)
(72) Inventor: YANG, Jiaxing, Beijing 102206 (CN); LI, Hai, Beijing 102206 (CN); WANG, Xiao, Beijing 102206 (CN)
(74) Representative: Murgitroyd & Company
(86) International application number: PCT/CN2024/116351
(87) International publication number: WO 2025/051095

(57) **Abstract**

The present disclose relates to the technical field of ophthalmic surgical microscopes and discloses a prism switching mechanism and an ophthalmic surgical microscope. The prism switching mechanism includes a housing assembly, a rotating block and a drive assembly, where: the rotating block is rotatably connected to the housing assembly, and provided circumferentially with a plurality of prisms along a rotation axis thereof; and the drive assembly is drivably connected to the rotating block, and configured to drive the rotating block to rotate, to switch the positions of the plurality of prisms in the horizontal direction.

## Description

### TECHNICAL FIELD

The present disclosure relates to the technical field of ophthalmic surgical microscopes, and specifically relates to a prism switching mechanism and an ophthalmic surgical microscope.

### BACKGROUND

The ophthalmic surgical microscope can be used for anterior segment imaging and posterior segment imaging (fundus imaging), which needs to implement switching between anterior segment imaging and posterior segment imaging in the optical system of the ophthalmic surgical microscope. For the existing switching structure, the imaging mode switching is accomplished by inverting a pentaprism and a roof prism in the vertical space. In the process of implementing embodiments of the present disclosure, the inventor has found that, when the prisms are inverted in the vertical space, it is required to overcome the gravity of the two prisms and the friction between the structures caused by gravity. Therefore, in the case of electric switching, this design has a high requirement for the output power of the motor; in the case of manual switching, this design is laborious for a user.

### SUMMARY

In view of the foregoing, the objective of the present disclosure is to provide a prism switching mechanism and an ophthalmic surgical microscope, to switch a plurality of prisms in the horizontal direction, without the need for overcoming the gravity of the prisms.

In order to accomplish the objective, the present disclosure adopts the following technical solution:
In an aspect, there is provided a prism switching mechanism, comprising:
a housing assembly;
a rotating block rotatably connected to the housing assembly, and provided circumferentially with a plurality of prisms along a rotation axis thereof; and
a drive assembly drivably connected to the rotating block, and configured to drive the rotating block to rotate, to switch the positions of the plurality of prisms in the horizontal direction.

As an optional technical solution of the prism switching mechanism, the housing assembly comprises a base and a support crossbar connected to an inner wall of the base, the rotating block on both ends in the vertical direction is provided with a first shaft portion and a second shaft portion, respectively, the support crossbar is provided with a first connecting hole, the first shaft portion is rotatably connected to the first connecting hole, and the second shaft portion is rotatably connected to the base.

As an optional technical solution of the prism switching mechanism, the drive assembly comprises a bracket, a motor, a first gear and a second gear, the first shaft portion extends through the first connecting hole, and the first gear is mounted on a part of the first shaft portion extending out of the first connecting hole; and the bracket is mounted in the base, the motor is mounted on the bracket, the second gear is mounted on an output end of the motor, and the second gear is drivably connected to the first gear.

As an optional technical solution of the prism switching mechanism, the drive assembly further comprises a third gear that is rotatably connected to the bracket, and is meshingly connected to the first gear and the second gear, respectively.

As an optional technical solution of the prism switching mechanism, the rotating block is provided circumferentially with two of the prisms along the rotation axis thereof, the two of the prisms are disposed on two sides of the rotating block, and one of the two of the prisms is a pentaprism while the other one is a roof prism.

As an optional technical solution of the prism switching mechanism, a first limiting portion 121 and a second limiting portion 122 are respectively disposed at both ends of the support crossbar 12, the rotating block is provided with an abutment portion, one side of the abutment portion abuts against the first limiting portion when the rotating block rotates forwardly relative to the support crossbar to a first working position, and the other side of the abutment portion abuts against the second limiting portion when the rotating block rotates reversely relative to the support crossbar to a second working position.

As an optional technical solution of the prism switching mechanism, one or both of the first limiting portion and the second limiting portion are threadedly connected with an adjusting screw rod, and the abutment portion can abut against an end face of the adjusting screw rod.

As an optional technical solution of the prism switching mechanism, the abutment portion is provided with a first mounting groove, a magnet is disposed in the first mounting groove, the adjusting screw rod is a magnetic member, and the magnet can be magnetically attracted to the adjusting screw rod.

As an optional technical solution of the prism switching mechanism, the prism switching mechanism further comprises a plurality of prism brackets that correspond to the plurality of the prisms, respectively, each of the prisms is mounted on one of the prism brackets, and the prism brackets are detachably connected to a sidewall of the rotating block.

As an optional technical solution of the prism switching mechanism, the prism brackets can be pitch - adjusted relative to the rotating block; and/or the prism brackets can be height - adjusted relative to the rotating block.

As an optional technical solution of the prism switching mechanism, the housing assembly further comprises an upper cover detachably connected to the base, the upper cover is provided with a second connecting hole, the first shaft portion extends through the first connecting hole and the second connecting hole, and a manual knob is mounted on a part of the first shaft portion extending out of the second connecting hole.

In a further aspect, there is provided an ophthalmic surgical microscope, comprising the prism switching mechanism of any of the embodiments of the present disclosure.

The present disclosure provides a prism switching mechanism and an ophthalmic surgical microscope. When the operating mode of the ophthalmic surgical microscope is to be switched, the positions of the prisms are switched by the prism switching mechanism, and the drive assembly drives the rotating block to rotate, to allow the positions of the plurality of prisms to be switched in the horizontal direction, thereby meeting different requirements for switching among different operating modes. Since the plurality of prisms are located in the circumferential direction of the rotating block along its rotation axis, there is no need for overcoming gravity of the prisms when these prisms are switched in the horizontal direction. Therefore, in the case of electric switching, this design has a low requirement for the output power of the motor; in the case of manual switching, this design saves effort of the user while improving the switching accuracy.

### BRIEF DESCRIPTION OF THE DRAWINGS

In order to make clearer the technical solution according to the embodiments of the present disclosure, brief introduction of the drawings required in the embodiments will be provided below. Apparently, the drawings described below only relate to some embodiments of the present disclosure, and the ordinary skill in the art could derive other drawings on the basis of those drawings, without doing creative work.
Fig. 1 exemplarily illustrates a schematic diagram of a structure of a prism switching mechanism according to an embodiment of the present disclosure;
Fig. 2 exemplarily illustrates a schematic diagram of a partial structure of a prism switching mechanism according to an embodiment of the present disclosure;
Fig. 3 exemplarily illustrates an exploded view of a structure of a prism switching mechanism according to an embodiment of the present disclosure;
Fig. 4 exemplarily illustrates an exploded view of a partial structure of a prism switching mechanism according to an embodiment of the present disclosure;
Fig. 5 exemplarily illustrates a top view of an anterior segment mode of a prism switching mechanism according to an embodiment of the present disclosure;
Fig. 6 exemplarily illustrates a top view of a posterior segment mode of a prism switching mechanism according to an embodiment of the present disclosure;
Fig. 7 exemplarily illustrates a sectional view of an anterior segment mode of a prism switching mechanism according to an embodiment of the present disclosure; and
Fig. 8 is an enlarged view of the A part in Fig. 7.

Reference signs therein are listed below:
1. housing assembly; 2. base; 111. boss; 112. front end; 113. lens; 12. support crossbar; 121. first limiting portion; 122. second limiting portion; 123. adjusting screw rod; 124. magnet; 125. first connecting hole; 126. set screw; 13. upper cover; 131. second connecting hole;
2. rotating block; 21. first shaft portion; 22. second shaft portion; 23. abutment portion; 24. first mounting groove; 25. second mounting groove;
3. drive assembly; 31. bracket; 32. motor; 33. first gear; 34. second gear; 35. third gear;
4. prism; 41. pentaprism; 42. roof prism;
5. prism bracket; 51. mounting opening; 52. fastening screw; 53. ball head locating pin; 54. adjustment groove;
6. manual knob.

### DETAILED DESCRIPTION OF EMBODIMENTS

Reference below will be made to the drawings and embodiments to further describe the present disclosure in detail. It would be appreciated that the specific embodiments described herein are provided only to make the present disclosure clear, rather than limit the present disclosure. It is also worth noting that, for ease of description, the drawings only depict a part of, not all, related structures of the present disclosure.

In the following description, unless specified or limited otherwise clearly, the terms "connecting," "connected," and "fixed" should be accorded with the broad explanation, which may be, for example, a fixed connection, a detachable connection or one piece, or which may be a mechanical connection or an electrical connection, or which may be directly connected or indirectly connected by an intermediate medium, or which may indicate internal communication between two elements or interaction between two elements. The ordinary skill in the art could understand the specific meanings of those terms in the present disclosure depending on the specific scenarios.

As used herein, unless specified or limited otherwise clearly, if a first feature is "above" or "below" a second feature, it may indicate that the first and second features are in direct contact with each other, or that they contact with each other via a further feature, rather than directly contact with each other. Moreover, if the first feature is "above," "on," and "over" the first feature, it may indicate that the first feature is directly or obliquely above the second feature, or may only indicate that the horizontal height of the first feature is greater than that of the second feature. If the first feature is "below," "under" and "beneath" the second feature, it may indicate that the first feature is directly or obliquely below the second feature, or may only indicate that the horizontal height of the first feature is less than that of the second feature.

As described in the embodiment, the terms "upper," "lower," "left," "right" and the like, which respectively indicate an orientation or position relationship, depend on the orientation or position relationships shown in the drawings and are used to facilitate description and simplify the operation, rather than indicate or imply that a concerned device or element should be constructed or operated at or in a specific orientation, which should not be construed as a limitation to the present disclosure. In addition, the terms "first," "second" and the like are only used for differentiation, not implying any particular meaning.

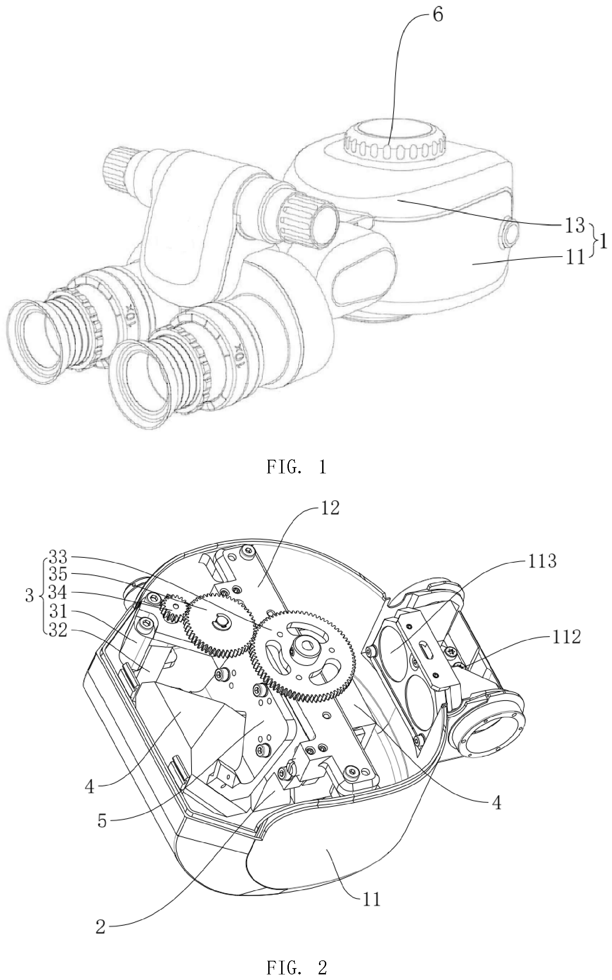

This embodiment provides an ophthalmic surgical microscope including a prism switching mechanism. As shown in Figs. 1 and 2, the prism switching mechanism includes a housing assembly 1, a rotating block 2 and a drive assembly 3, where: the rotating block 2 is rotatably connected to the housing assembly 1, and provided circumferentially with a plurality of prisms 4 along its rotation axis ; the drive assembly 3 is drivably connected to the rotating block 2, and configured to drive the rotating block 2 to rotate such that positions of the plurality of prisms 4 can be switched in the horizontal direction. When the operating mode of the ophthalmic surgical microscope is to be switched, the positions of the prisms 4 are switched by the prism switching mechanism, and the drive assembly 3 drives the rotating block 2 to rotate, where the rotation axis of the rotating block 2 is arranged vertically to allow the positions of the plurality of prisms 4 to be switched in the horizontal direction, thereby meeting different requirements for switching among different operating modes. Since the plurality of prisms 4 are located in the circumferential direction of the rotating block 2 along its rotation axis, there is no need for overcoming gravity of the prisms 4 when these prisms 4 are switched in the horizontal direction. Therefore, in the case of electric switching, this design has a low requirement for the output power of the motor; in the case of manual switching, this design saves effort of the user while improving the switching accuracy.

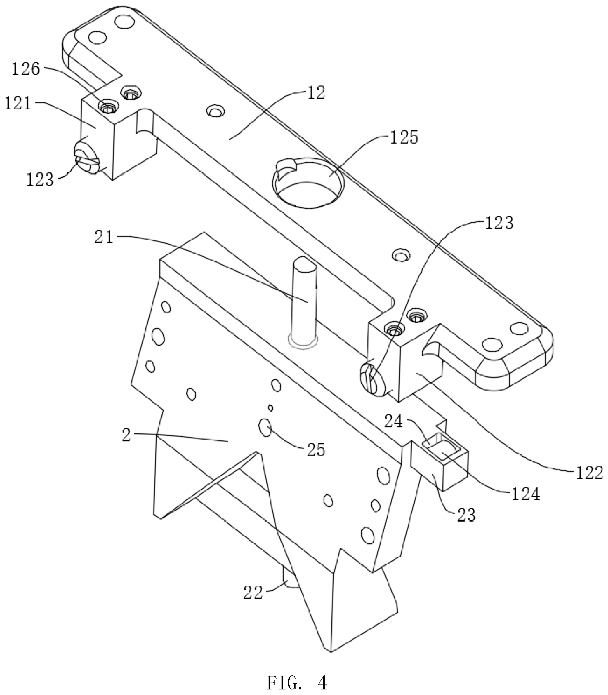

In the embodiment, as shown in Figs. 2-4, the housing assembly 1 includes a base 11 and a support crossbar 12 that is connected to the inner wall of the base 11; the rotating block 2 on both ends in the vertical direction is provided with a first shaft portion 21 and a second shaft portion 22, respectively; the support crossbar 12 is provided with a first connecting hole 125; the first shaft portion 21 is rotatably connected to the first connecting hole 125; the second shaft portion 22 is rotatably connected to the base 11; in this way, the rotating block 2 is rotatably connected to the housing assembly 1. Wherein, the base 11 on the inner wall is provided with two bosses 111, both ends of the support crossbar 12 are lapped to the two bosses 111, and both ends of the support crossbar 12 are connected to the two bosses 111 by screws, respectively, thus improving the connection stability.

Further, the drive assembly 3 includes a bracket 31, a motor 32, a first gear 33 and a second gear 34, the first shaft portion 21 extends through the first connecting hole 125, and the first portion 21 at a part thereof extending out of the first connecting hole 125 has the first gear 33 mounted thereon; the bracket 31 is mounted in the base 11, the motor 32 is mounted on the bracket 31, and the motor 32 at the output end has a second gear 34 mounted thereon, which is drivably connected to the first gear 33. The motor 32 drives the second gear 34 to rotate, and the second gear 34 drives the first gear 33 and the rotating block 2 to rotate, such that the drive assembly 3 can drive the rotating block 2 to rotate, to thus drive the prisms 4 to switch.

Optionally, the drive assembly 3 further includes a third gear 35 that is rotatably connected to the bracket 31, and is meshingly connected to the first gear 33 and the second gear 34. By means of the third gear 35, the second gear 34 indirectly drives the first gear 33 to rotate, which can reduce the size of the second gear 34 and thus facilitates the spatial layout.

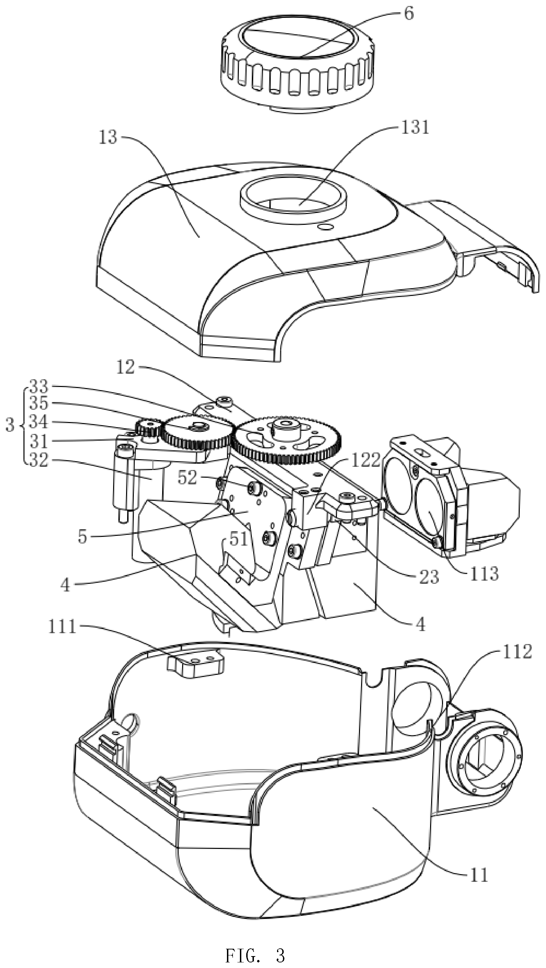

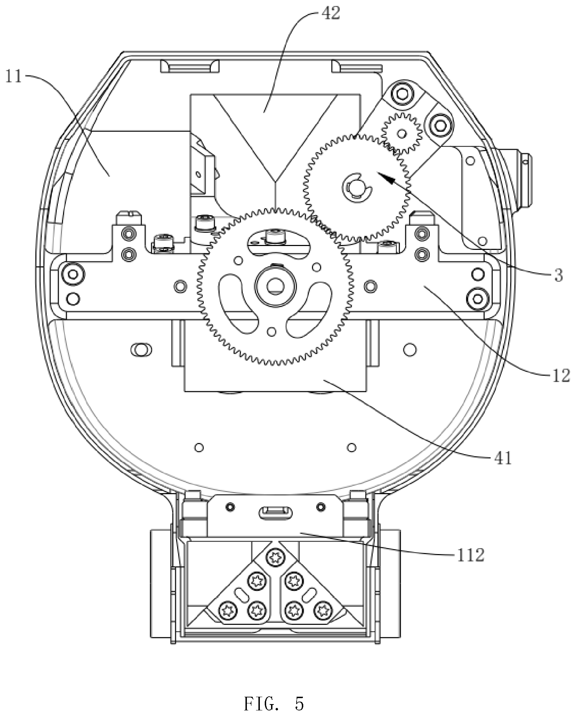

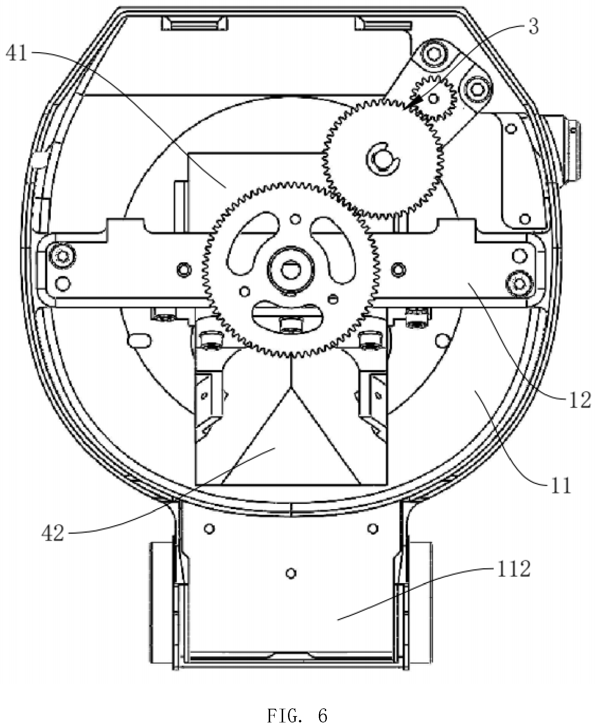

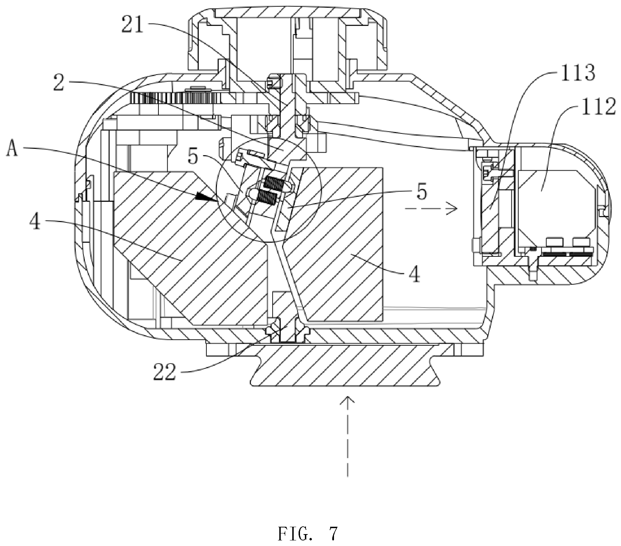

In the embodiment, as shown in Fig. 3, the rotating block 2 is provided circumferentially with two prisms 4 along its rotation axis, where the two prisms 4 are disposed on two sides of the rotating block 2, and one of the two prisms 4 is a pentaprism 41 while the other is a roof prism 42. Referring to Figs. 5 and 7, the base 11 is provided with a front end 112 in which a lens 113 is disposed, and the pentaprism 41 is rotated to a side close to the lens 113 such that the light incident surface of the pentaprism 41 is perpendicular to the incident light, and the light outgoing surface of the pentaprism 41 is perpendicular to the axis of the lens 113, to thus switch the ophthalmic surgical microscope to the anterior segment mode. Referring to Fig. 6, the roof prism 42 is rotated to the side close to the lens 113 such that the light incident surface of the roof prism 42 is perpendicular to the incident light, and the light outgoing surface of the roof prism 42 is perpendicular to the axis of the lens 113, to thus switch the ophthalmic surgical microscope to the posterior segment mode.

Optionally, a first limiting portion 121 and a second limiting portion 122 are respectively disposed at both ends of the support crossbar 12, and the rotating block 2 is provided with an abutment portion 23, where one side of the abutment portion 23 abuts against the first limiting portion 121 when the rotating block 2 rotates forwardly relative to the support crossbar 12 to the first working position, and the other side of the abutment portion 23 abuts against the second limiting portion 122 when the rotating block 2 rotates reversely relative to the support crossbar 12 to the second working position. With the first limiting portion 121 and the second limit portion 122, the position of the rotating block 2 can be positioned, to ensure that the prisms 4 can be rotated to preset positions, and improve the switching accuracy. In this embodiment, since the pentaprism 41 and the roof prism 42 are disposed on two sides of the rotating block 2, the rotation angle for switching between the first working position and the second working position is 180°.

Optionally, referring to Figs. 2 and 3, one or both of the first limiting portion 121 and the second limiting portion 122 are threadedly connected with an adjusting screw rod , and the abutment portion 23 can abut against the end face of the adjusting screw rod 123. In the embodiment, both the first limiting portion 121 and the second limiting portion 122 are threadedly connected with the adjusting screw rod 123, and the screwing depth of the threaded rod 123 relative to the first limiting portion 121 and the second limiting portion 122 is controlled and adjusted, to adjust the rotation angle of the rotating block 2 and further ensure that the prisms 4 can be rotated to the preset position, thus achieving a good control performance. Wherein, the first limiting portion 121 and the second limiting portion 122 are provided with a first threaded hole; the adjusting screw rod 123 is threadedly connected to the first threaded hole; optionally, the first threaded hole on the side wall is provided with a second threaded hole; the setscrew 126 is threadedly connected to the second threaded hole; when the adjusting screw rod 123 is adjusted to the preset position, the setscrew 126 abuts against the sidewall of the adjusting screw rod 123, to prevent the adjusting screw rod 123 from moving relative to the first limiting portion 121 and the second limiting portion 122, and thus improve the structure reliability.

When the drive assembly 3 drives the rotating block 2 to rotate, the abutment portion 23 may hit and rebound from the end face of the adjustment threaded screw 123 due to a too great force, thus making it impossible to ensure that the abutment portion 23 can be fully fit with the end face of the adjusting screw rod 123. In order to solve the above problem, the abutment portion 23 is provided with a first mounting groove 24, a magnet 124 is disposed in the first mounting groove 24, the adjusting screw rod 123 is a magnet member, and the magnet 124 can be magnetically attracted to the adjusting screw rod 123. When the abutment portion 23 abuts against the end face of the adjusting screw rod 123, the magnet 124 is magnetically attracted to the adjusting screw rod 123, to ensure that the abutment portion 23 can be fully fit with the end face of the adjusting screw rod 123, to prevent the rotation block 2 from vibrating and shaking, and to improve the repeated positioning accuracy. Wherein, the adjusting screw rod 123 may be formed of Martensitic stainless steel.

Alternatively, when the drive assembly 3 drives the rotating block 2 to rotate, the abutment portion 23 may not abut against the end face of the adjusting screw rod 123 due to a too small force. In the case, the magnet 124 and the adjusting screw rod 123 are magnetically attracted, such that the abutment portion 23 can be attracted to the end face of the adjusting screw rod 123, to assist the abutment portion 23 in abutting against the adjusting screw rod 123.

In the embodiment, referring to Fig. 3, the prism switching mechanism further includes a plurality of prism brackets 5 that correspond to the plurality of prisms 4, respectively; each prism 4 is mounted on a prism bracket 5; the prism brackets 5 are detachably connected to the sidewall of the rotating block 2; in this way, the prisms 4 are mounted on the sidewall of the rotating block 2.

It is worth noting that the mounting angle of the prism 4 can be adaptively adjusted as actually required. In this embodiment, the prism brackets 5 can be pitch - adjusted relative to the rotating block 2; and/or the prism brackets 5 can be height - adjusted relative to the rotating block 2.

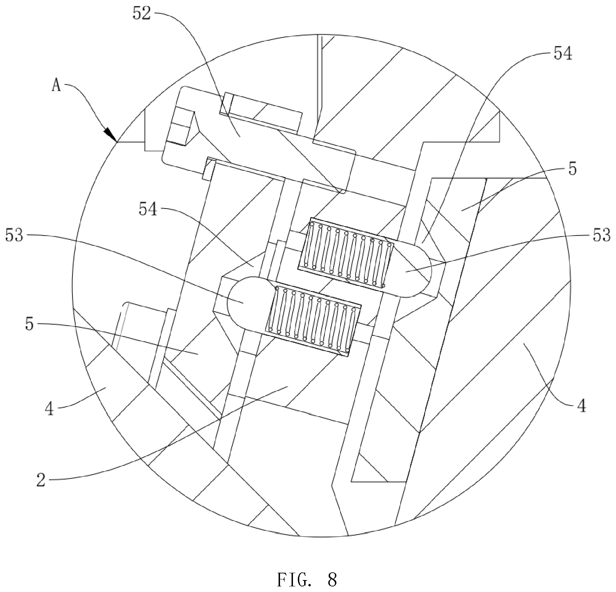

In the embodiment, referring to Figs. 3, 7 and 8, the prism bracket 5 is provided thereon with a mounting opening 51, the prism 4 can be embedded in the mounting opening 51, and the prism 4 is glued to the mounting opening 51. The prism bracket 5 is provided with at least three through holes; the rotating block 2 on the sidewall is provided with at least three third threaded holes; the three through holes correspond to the third threaded holes, respectively; the fastening screw 52 extends through the through hole and is threadedly connected to the third threaded hole; in this way, the prism bracket 5 is detachably connected to the sidewall of the rotating block 2. Since the adjustment screw and the through hole are in clearance fit, the prism bracket 5 can move in the vertical direction relative to the adjustment screw, such that the prism bracket 5 can be height - adjusted relative to the rotating block 2.

Optionally, the prism bracket 5 on a side close to the rotating block 2 is provided with an adjustment groove 54; the rotating block 2 on the sidewall is provided with a second mounting groove 25; a ball head locating pin 53 is mounted within the second mounting groove 25; the ball head locating pin 53 abuts against the groove bottom of the adjustment groove 54; the ball head locating pin 53 causes a clearance to be formed between the part of the prism bracket 5, on which the adjustment groove 54 is arranged, and the sidewall of the rotating block 2, where the clearance may be, for example, 1 mm; by adjusting the three adjustment screws to different screwing depths, the prism bracket 5 can be pitch - adjusted relative to the rotating block 2.

Further, as shown in Figs. 1 and 3, the housing assembly 1 further includes an upper cover 13 that is detachably connected to the base 11 and provided with a second connecting hole 131; the first shaft portion 21 extends through the first connecting hole 125 and the second connecting hole 131; and a manual knob 6 is mounted on the part of the first shaft portion 21 extending out of the second connecting hole 131.

In this embodiment, the pentaprism 41 and the roof prism 42 are mounted on the corresponding prism brackets 5; the prism brackets 5 are connected to the sidewall of the rotating block 2; the drive assembly 3 is used to drive the prism 4 to switch, to accomplish that the motor drives the pentaprism 41 and the roof prism 42 to switch freely in the optical system. Alternatively, by operating the manual knob 6 to rotate, the rotating block 2 and the prism 4 are driven to rotate, thus causing the prisms 4 to be manually switched.

The above are only preferred embodiments of the present disclosure and technical principles applied therein. It would be appreciated by those skilled in the art that the present disclosure is not limited to the specific embodiments described herein, and various obvious changes, rearrangements and substitutions can be made without departing from the scope of protection of the present disclosure. Therefore, although described in detail with reference to the above embodiments, the present disclosure is not limited to those embodiments and may cover more other equivalent embodiments without departing from the conception of the present disclosure. The scope of the present disclosure is determined by the appended claims.

## Claims

1. A prism switching mechanism, comprising:
a housing assembly (1);
a rotating block (2) rotatably connected to the housing assembly (1), and provided circumferentially with a plurality of prisms (4) along a rotation axis thereof; and
a drive assembly (3) drivably connected to the rotating block (2), and configured to drive the rotating block (2) to rotate, to switch the positions of the plurality of prisms (4) in the horizontal direction.

2. The prism switching mechanism of claim 1, wherein the housing assembly (1) comprises a base (11) and a support crossbar (12) connected to an inner wall of the base (11), the rotating block (2) on both ends in the vertical direction is provided with a first shaft portion (21) and a second shaft portion (22), respectively, the support crossbar (12) is provided with a first connecting hole (125), the first shaft portion (21) is rotatably connected to the first connecting hole (125), and the second shaft portion (22) is rotatably connected to the base (11).

3. The prism switching mechanism of claim 2, wherein the drive assembly (3) comprises a bracket (31), a motor (32), a first gear (33) and a second gear (34), the first shaft portion (21) extends through the first connecting hole (125), and the first gear (33) is mounted on a part of the first shaft portion (21) extending out of the first connecting hole (125); and wherein the bracket (31) is mounted in the base (11), the motor (32) is mounted on the bracket (31), the second gear (34) is mounted on an output end of the motor (32), and the second gear (34) is drivably connected to the first gear (33).

4. The prism switching mechanism of claim 3, wherein the drive assembly (3) further comprises a third gear (35) that is rotatably connected to the bracket (31), and meshed with the first gear (33) and the second gear (34), respectively.

5. The prism switching mechanism of claim 2, wherein the rotating block (2) is provided circumferentially with two of the prisms (4) along the rotation axis thereof, the two of the prisms (4) are disposed on two sides of the rotating block (2), and one of the two of the prisms (4) is a pentaprism (41) while the other one is a roof prism (42).

6. The prism switching mechanism of claim 5, wherein both ends of the support crossbar (12) are provided with a first limiting portion (121) and a second limiting portion (122), respectively, the rotating block (2) is provided with an abutment portion (23), one side of the abutment portion (23) abuts against the first limiting portion (121) when the rotating block (2) rotates forwardly relative to the support crossbar (12) to a first working position, and the other side of the abutment portion (23) abuts against the second limiting portion (122) when the rotating block (2) rotates reversely relative to the support crossbar (12) to a second working position.

7. The prism switching mechanism of claim 6, wherein one or both of the first limiting portion (121) and the second limiting portion (122) are threadedly connected with an adjusting screw rod (123), and the abutment portion (23) can abut against an end face of the adjusting screw rod (123).

8. The prism switching mechanism of claim 7, wherein the abutment portion (23) is provided with a first mounting groove (24), a magnet (124) is disposed in the first mounting groove (24), the adjusting screw rod (123) is a magnetic member, and the magnet (124) can be magnetically attracted to the adjusting screw rod (123).

9. The prism switching mechanism of claim 1, further comprising a plurality of prism brackets (5) that correspond to the plurality of the prisms (4), respectively, each of the prisms (4) is mounted on one of the prism brackets (5), and the prism brackets (5) are detachably connected to a sidewall of the rotating block (2).

10. The prism switching mechanism of claim 9, wherein,
the prism brackets (5) can be pitch - adjusted relative to the rotating block (2); and/or
the prism brackets (5) can be height - adjusted relative to the rotating block (2).

11. The prism switching mechanism of claim 2, wherein the housing assembly (1) further comprises an upper cover (13) detachably connected to the base (11), the upper cover (13) is provided with a second connecting hole (131), the first shaft portion (21) extends through the first connecting hole (125) and the second connecting hole (131), and a manual knob (6) is mounted on a part of the first shaft portion (21) extending out of the second connecting hole (131).

12. An ophthalmic surgical microscope, comprising the prism switching mechanism of any of claims 1-11.
